# FASCICULE DE BREVET EUROPEEN

(11) **EP 4 367 124 B1**
(45) Date de publication et mention de la délivrance du brevet: **13.08.2025**
(21) Numéro de dépôt: 22744691.1
(22) Date de dépôt: 05.07.2022
(51) Int. Cl.: C07H 1/00, C07H 17/07

(54) **PROCÉDÉ DE PRÉPARATION DE DIOSMINE ET DE FRACTION FLAVONOÏQUE**
VERFAHREN ZUR HERSTELLUNG VON DIOSMIN UND FLAVONOIDFRAKTION
METHOD FOR PREPARING DIOSMIN AND FLAVONOID FRACTION

(30) Priorité: 06.07.2021 EP 21305932
(43) Date de publication de la demande: 15.05.2024
(73) Titulaire: Les Laboratoires Servier, 92284 Suresnes Cedex (FR)
(72) Inventeur: SARI, Ozkan, 93300 Aubervilliers (FR); SCHIAVI, Bruno, 76210 Gruchet le Valasse (FR); LAVAL, Stéphane, 21000 Dijon (FR); RASSON, Corentin, 1300 Wavre (BE)
(74) Mandataire: Giudicelli, Cathy
(86) Numéro de dépôt international: PCT/EP2022/068596
(87) Numéro de publication internationale: WO 2023/280857

(56) Documents cités:
- EP-A1- 3 321 273
- WO-A1-2016/124585
- FR-A1- 2 311 028
- FR-A1- 2 782 518

## Description

La présente invention concerne un procédé de préparation de diosmine.

La diosmine est le composé de formule (I) :

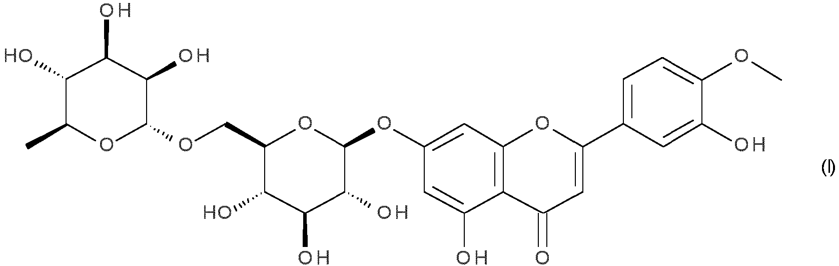

La diosmine est synthétisée par oxydation de l'hespéridine. L'hespéridine est le composé de formule (II) :

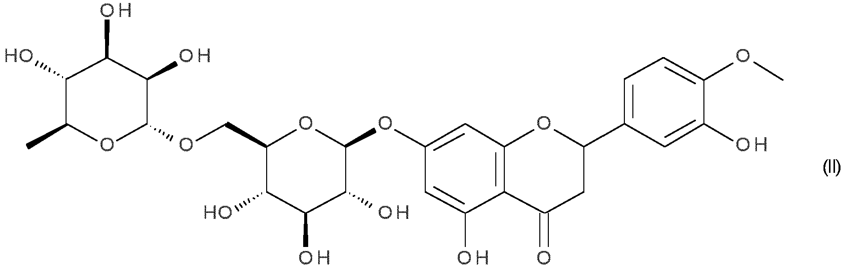

L'hespéridine est obtenue à partir de substances naturelles (orangettes). La diversité des orangettes utilisées conduit à des hespéridines de pureté inégale, contenant d'autres flavonoïdes avec des teneurs variables. Par exemple, l'hespéridine peut contenir jusqu'à 4% d'isonaringine, qui se transforme en isorhoifoline par oxydation.

La diosmine contient donc en général d'autres flavonoïdes, dont certains proviennent de l'oxydation des flavonoïdes présents dans l'hespéridine de départ, et d'autres sont des produits secondaires de réaction.

Les spécifications imposées par la Pharmacopée Européenne pour la diosmine sont les suivantes:

| Substances | Spécifications diosmine (Pharmacopée Européenne) |
|---|---|
| Diosmine | 90,0 à 102,0 % |
| Hespéridine | < 4,0% |
| Diosmétine | < 2,0 % |
| Isorhoifoline | < 3,0 % |
| Linarine | < 3,0 % |
| 6-Iododiosmine | < 0,6 % |

On entend par Diosmine Pharmacopée une diosmine satisfaisant aux spécifications ci-dessus.

La diosmine est également le composant majoritaire de la fraction flavonoïque purifiée micronisée, ou FFPM (Daflon^{®}).

Par fraction flavonoïque, on entend un principe actif comprenant environ 90% de diosmine, en mélange avec de l'hespéridine, de la diosmétine, de la linarine et de l'isorhoifoline.

La fraction flavonoïque selon la présente invention comprend préférentiellement de 86,0 à 95,0% de diosmine, de 2,5 à 6,0% d'hespéridine, de 0,2 à 2% de diosmétine, de 0,8 à 3,5% de linarine et de 0,8 à 3,5% d'isorhoifoline.

En outre, la fraction flavonoïque selon la présente invention peut contenir d'autres flavonoïdes dérivés de l'hespéridine dont la teneur totale ne dépasse pas 1,0%.

La Diosmine Pharmacopée et la fraction flavonoïque purifiée micronisée sont utilisées dans le traitement des maladies veineuses, comme l'insuffisance veineuse chronique ou les maladies hémorroïdales.

Compte tenu de leur intérêt pharmaceutique, il est primordial de les obtenir avec un excellent rendement et une bonne pureté, même en partant d'une hespéridine ayant une pureté de l'ordre de 90 à 95%.

En particulier, il est primordial que la diosmine obtenue, qu'elle soit sous forme de Diosmine Pharmacopée ou de fraction flavonoïque, contienne moins de 0,6% de 6-iododiosmine.

Des procédés de préparation de la diosmine à partir d'hespéridine ont été décrits dans la littérature.

Les demandes de brevets FR2311028 et WO2016/124585 décrivent l'obtention de la diosmine par acétylation de l'hespéridine, oxydation de l'hespéridine acétylée en diosmine acétylée puis désacétylation.

Ce procédé n'est pas idéal, car le rendement n'est que de 65% et de 77% respectivement. De plus, il comprend plusieurs étapes.

Les demandes de brevet EP3321273 et FR2782518 décrivent l'obtention de la diosmine par oxydation de l'hespéridine par de l'iode en une seule étape, dans un solvant amide (EP3321273) ou dans la pyridine (FR2782518).

Cependant, l'utilisation d'iode en quantité stochiométrique est problématique à l'échelle industrielle.

Le problème de la présente invention était d'obtenir la Diosmine Pharmacopée ou la fraction flavonoïque à partir d'hespéridine en une seule étape avant isolement (sans passer par l'étape d'acétylation de l'hespéridine et de désacétylation de la diosmine acétylée), avec un excellent rendement, tout en minimisant la teneur en 6-iododiosmine dans le produit obtenu.

Plus spécifiquement, la présente invention concerne un procédé de préparation de Diosmine Pharmacopée ou de fraction flavonoïque par oxydation de l'hespéridine en diosmine par un couple oxydant donneur d'iode, à une température de 80 à 120°C, dans un mélange de solvant aprotique polaire et d'acide acétique, suivie de l'isolement par ajout d'eau, filtration, rinçage et séchage.

Selon un mode de réalisation de la présente invention, la diosmine est obtenue sous la forme de Diosmine Pharmacopée.

Selon un autre mode de réalisation, la diosmine est obtenue sous la forme de fraction flavonoïque comprenant de 86,0 à 95,0% de diosmine, de 2,5 à 6,0% d'hespéridine, de 0,2 à 2% de diosmétine, de 0,8 à 3,5% de linarine et de 0,8 à 3,5% d'isorhoifoline.

En outre, la fraction flavonoïque selon la présente invention peut contenir d'autres flavonoïdes dérivés de l'hespéridine dont la teneur totale ne dépasse pas 1,0%.

Le couple oxydant donneur d'iode est préférentiellement choisi parmi NaI/H₂O₂, KI/H₂O₂, TBAI/H₂O₂ et NaI/I₂ /H₂O₂.

La quantité de donneur d'iode NaI, KI ou TBAI est préférentiellement de 0,4 à 0,8 équivalent molaire par rapport à l'hespéridine engagée.

La quantité d'eau oxygénée est préférentiellement de 0,9 à 1,1 équivalent molaire par rapport à l'hespéridine engagée.

Lorsque NaI/I₂/H₂O₂ est utilisé, le rapport molaire NaI/I₂ est préférentiellement d'environ 9/1.

Parmi les solvants aprotiques polaires utilisables dans le procédé selon l'invention, on peut citer à titre d'exemple le diméthylsulfoxyde, la N-méthylpyrrolidone et le diméthylacétamide.

Le solvant aprotique polaire préféré est le diméthylsulfoxyde.

Lorsque le solvant aprotique polaire est le diméthylsulfoxyde, des impuretés S-méthylées peuvent se former au cours du procédé. Il est important de minimiser la teneur de ces impuretés.

Selon un mode de réalisation de la présente invention, la diosmine brute obtenue est purifiée par un traitement base/acide : passage en solution dans l'eau en présence d'une base telle que l'hydroxyde de sodium, puis précipitation par salification avec un acide tel que l'acide sulfurique, filtration, rinçage et séchage.

Selon un autre mode de réalisation de la présente invention, la diosmine brute est d'abord réempâtée dans un solvant organique tel que le diméthylsulfoxyde, la N-butylpyrrolidone, la pyridine, le sulfolane, le carbonate de diméthyle ou le carbonate de propylène, ou dans un mélange de solvant organique et d'eau tel qu'un mélange de pyridine et d'eau, puis filtrée et rincée par de l'eau, avant de subir un traitement base/acide.

Selon un autre mode de réalisation de la présente invention, la diosmine obtenue est purifiée par un traitement base/acide en présence d'eau oxygénée.

Les exemples suivants illustrent l'invention.

La pureté de l'hespéridine utilisée dans les exemples était de 93,6 %. L'utilisation d'une hespéridine de haut degré de pureté permet d'obtenir de la Diosmine Pharmacopée.

### Abréviations :

- DMSO: diméthylsulfoxyde
- éq.: équivalents molaires (par rapport à l'hespéridine)
- HPLC: High Performance Liquid Chromatography (Chromatographie en phase liquide à haute performance)
- m/m: rapport exprimé en masse/masse
- NBP: N-Butyl-Pyrrolidone
- ND: Non détectée
- TBAI: Iodure de tétrabutyl ammonium
- tr/min: tours par minute
- vol: équivalent volumique (exprimé en L/kg du flavonoïde engagé)

### EXEMPLE 1 : Diosmine brute

Dans un réacteur de 6L, introduire l'hespéridine (800 g, 1,31 mol, 1,0 éq.) et l'iodure de sodium (123,7 g, 0,82 mol, 0,63 éq).

Ajouter ensuite le DMSO (3,1 vol, soit 2,48 L) et l'acide acétique (0,5 vol, soit 0,40 L). Agiter à 250 tr/min (ancre) puis chauffer à 97 °C (Tₘₐₛₛₑ).

A 97 °C, ajouter la solution aqueuse de H₂O₂ 3.5M (367 mL, 0,98 éq.).

A la fin de la coulée, le milieu réactionnel hétérogène est laissé à agiter à 97°C pendant 15 min puis ramené à 25°C.

A 25°C, de l'eau (1,5 vol, soit 1,2 L) est ajoutée (exotherme de 12°C), puis le milieu est filtré sur un fritté de porosité 3.

Le gâteau est rincé avec de l'eau (2x 2,5 vol, soit 2x 2,0 L)

Le gâteau obtenu est essoré sur filtre. La diosmine brute ainsi obtenue peut être engagée directement dans l'étape suivante.

Alternativement, elle peut être isolée et séchée dans une étuve ventilée à 80°C pendant une nuit. La diosmine est alors obtenue avec un rendement de 94% et une teneur en 6-iodo-diosmine de 0,52 %.

### EXEMPLE 2 : Traitement base/acide et micronisation

Transférer la diosmine brute non séchée obtenue à l'Exemple 1 dans un réacteur de 6L.

Ajouter l'eau (5,0 vol par rapport à l'hespéridine engagée dans l'Exemple 1, soit 4,0 L) et l'hydroxyde de sodium (611 g, 4,58 mol, 3,5 éq.).

Agiter à 25°C jusqu'à dissolution complète (typiquement 1h).

Clarifier le milieu sur un pad de Clarcel^{®} puis transférer le filtrat dans un réacteur de 6L.

Refroidir la solution à 10°C puis ajouter lentement l'acide sulfurique 37% (typiquement 611 g, 2,30 mol) sans dépasser 13°C (Tₘₐₛₛₑ) et jusqu'à obtention de pH=3.

Agiter 45 minutes à 10°C pour finaliser la précipitation.

Filtrer le milieu sur un fritté de porosité 3.

Rincer le gâteau avec de l'eau (2x 2,5 vol, soit 2x 2,0 L).

Le produit est séché dans une étuve ventilée à 80°C pendant une nuit. Le produit obtenu peut être engagé dans une étape de retraitement en présence d'eau oxygénée.

Alternativement, le solide obtenu peut être micronisé.

On obtient alors la diosmine sous la forme de fraction flavonoïque micronisée avec un rendement de 92% à partir de l'hespéridine et une teneur en 6-iodo-diosmine de 0,5 %.

| Substances | Pourcentage dans le produit de l'Exemple 2 |
|---|---|
| Diosmine | 87,8% |
| 6-Iododiosmine | 0,5 % |

### EXEMPLE 3 : Retraitement base/acide en présence d'eau oxygénée

Le retraitement base/acide en présence d'eau oxygénée permet d'éliminer les éventuelles impuretés S-méthylées.

Dans un erlenmeyer de 250 mL, introduire la diosmine obtenue avant micronisation à l'Exemple 2 (10,0 g, 16,4 mmol).

Ajouter ensuite l'eau (5,0 vol, soit 50 mL) et la soude (7,67 g, 3,5 éq.).

Agiter jusqu'à dissolution complète.

Ajouter l'eau oxygénée 35% (0,1 ou 0,2 éq.) en une portion puis agiter à température ambiante 30 min à 3h.

Refroidir à 10°C puis ajouter l'acide sulfurique (7,40 g, 1,7 éq.) jusqu'à pH=3

Filtrer le milieu sur un fritté de porosité 3.

Le gâteau est rincé à l'eau (2x 2,0 vol, soit 2x 10 mL).

Le solide est ensuite séché durant une nuit à 80°C dans une étuve ventilée.

On obtient la diosmine sous la forme de fraction flavonoïque.

| Substances | 0,1 éq. H₂O₂ 30mn | 0,2 éq. H₂O₂ 3h |
|---|---|---|
| Diosmine | 88,1 % | 88,6 % |
| 6-Iododiosmine | 0,4 % | 0,4% |

### EXEMPLE 4 : Réempâtage pyridine/eau puis traitement base/acide

La diosmine brute isolée et séchée obtenue dans l'Exemple 1 a été réempâtée dans le système binaire pyridine /H₂O 6/1 (rapport volumique), à 25°C, pendant 90 min. Ce réempatage permet de réduire la teneur en 6-iodo-diosmine et d'éliminer les éventuelles impuretés S-méthylées.

Transférer la diosmine brute (100g, poids sec, 164,3 mmol) obtenue à l'Exemple 1 dans un réacteur de 1L.

Ajouter l'eau (1,0 vol, soit 100 mL) et la pyridine (6 vol, soit 600 mL).

Agiter à 25°C pendant 90 minutes.

Filtrer le milieu sur fritté porosité 3.

Rincer le gâteau avec de l'eau (2x 2,5 vol, soit 2x 250 mL).

Essorer le solide sur filtre et transférer le produit sans séchage additionnel dans un réacteur de 1L.

Ajouter l'eau (5,0 vol, soit 500 mL) et l'hydroxyde de sodium (76,7 g, 575,1 mol, 3,5 éq.).

Agiter à 25°C jusqu'à dissolution complète (typiquement 1h).

Clarifier le milieu sur un pad de Clarcel^{®} puis transférer le filtrat dans un réacteur de 1L.

Refroidir la solution à 10°C puis ajouter lentement l'acide sulfurique 37% (typiquement 76,7 g, 289,3 mmol) sans dépasser 13°C (Tₘₐₛₛₑ) et jusqu'à obtention de pH=3.

Agiter 45 minutes à 10°C pour finaliser la précipitation.

Filtrer le milieu sur un fritté de porosité 3.

Rincer le gâteau avec de l'eau (2x 2,5 vol, soit 2x 250 mL).

Le produit est séché dans une étuve ventilée à 80°C pendant une nuit.

On obtient la diosmine sous la forme de fraction flavonoïque avec une pureté supérieure à 88%, un rendement de 88% à partir de l'hespéridine et une teneur en 6-iodo-diosmine de 0,4 %.

| Substances | |
|---|---|
| Diosmine | 88,4 % |
| 6-Iododiosmine | 0,4 % |
| Impuretés S-méthylées | Non détectées |

### EXEMPLE 5 : Réempâtage dans d'autres solvants.

La diosmine brute utilisée a été obtenue dans les conditions de l'Exemple 1.

### Réempâtage DMSO:

Dans un tricol de 100mL, introduire la diosmine (5,0 g) et le DMSO (3 vol) puis chauffer à 80°C jusqu'à solubilisation complète.

Laisser refroidir à 25°C puis additionner l'eau (3 vol). Filtrer sur fritté porosité 3, rincer le gâteau avec de l'eau (2 x 2,5 vol) puis sécher en étuve ventilée une nuit à 80°C.

On obtient la diosmine sous forme de fraction flavonoïque (4,9 g).

### Réempâtage sulfolane:

Dans un tricol de 100mL, introduire la diosmine (5,0 g) et le sulfolane (7 vol) puis agiter à température ambiante pendant 1h.

Filtrer sur fritté porosité 3. Rincer le gâteau avec de l'eau (2 x 2,5 vol) puis sécher en étuve ventilée une nuit à 80°C.

On obtient la diosmine sous forme de fraction flavonoïque (5,0 g).

### Réempâtage carbonate de diméthyle:

Dans un tricol de 100mL, introduire la diosmine (5,0 g) et le carbonate de diméthyle (7 vol) puis agiter à température ambiante pendant 1h.

Filtrer sur fritté porosité 3. Rincer le gâteau avec de l'eau (2 x 2,5 vol) puis sécher en étuve ventilée 2 jours à 50°C.

On obtient la diosmine sous forme de fraction flavonoïque (4,9 g).

### Réempâtage carbonate de propylène:

Dans un tricol de 100mL, introduire la diosmine (5,0 g) et le carbonate de propylène (7 vol) puis agiter à température ambiante pendant 1h.

Filtrer sur fritté porosité 3. Rincer le gâteau avec de l'eau (2 x 2,5 vol) puis sécher en étuve ventilée 2 jours à 50°C.

On obtient la diosmine sous forme de fraction flavonoïque (4,8 g).

### Réempâtage NBP:

Dans un tricol de 100mL, introduire la diosmine (5,0 g) et la N-butylpyrrolidone (7 vol) puis agiter à température ambiante pendant 1h.

Filtrer sur fritté porosité 3. Rincer le gâteau avec de l'eau (2 x 2,5 vol) puis sécher en étuve ventilée une nuit à 80°C.

On obtient la diosmine sous forme de fraction flavonoïque (4,8 g).

| Substances | Diosmine brute | Purification par réempâtage | | | | |
|---|---|---|---|---|---|---|
| | Avant réempâtage | DMSO | Sulfolane | Carbonate de diméthyle | Carbonate de propylène | NBP |
| Diosmine | 88,2% | 86,8% | 86,9% | 86,9% | 86,9% | 87,3% |
| 6-Iododiosmine | 0,5% | 0,5% | 0,5% | 0,5% | 0,5% | 0,4% |
| Impuretés S-méthylées | <0,1% | ND | ND | ND | ND | ND |

## Revendications

1. Procédé de préparation de diosmine par oxydation de l'hespéridine par un couple oxydant donneur d'iode, à une température de 80 à 120°C, dans un mélange de solvant aprotique polaire et d'acide acétique, suivie de l'isolement par ajout d'eau, filtration, rinçage et séchage.

2. Procédé selon la revendication 1, dans lequel la diosmine obtenue est sous la forme de Diosmine Pharmacopée, comprenant de 90,0 à 102,0% de diosmine, moins de 4,0% d'hespéridine, moins de 2,0% de diosmétine, moins de 3,0% d'isorhoifoline, moins de 3,0% de linarine et moins de 0,6% de 6-iododiosmine.

3. Procédé selon la revendication 1, dans lequel la diosmine obtenue est sous la forme de fraction flavonoïque comprenant de 86,0 à 95,0% de diosmine, de 2,5 à 6,0% d'hespéridine, de 0,2 à 2% de diosmétine, de 0,8 à 3,5% de linarine et de 0,8 à 3,5% d'isorhoifoline.

4. Procédé selon la revendication 1 ou 3, dans lequel la diosmine obtenue contient moins de 0,6% de 6-iododiosmine.

5. Procédé selon l'une quelconque des revendications 1 à 4, dans lequel le couple oxydant donneur d'iode est choisi parmi NaI/H₂O₂, KI/H₂O₂, TBAI/H₂O₂ et NaI/I₂/H₂O₂.

6. Procédé selon la revendication 5, dans lequel le couple oxydant donneur d'iode est NaI/H₂O₂.

7. Procédé selon la revendication 6, dans lequel la quantité de NaI est de 0,4 à 0,8 équivalent molaire par rapport à l'hespéridine engagée.

8. Procédé selon l'une quelconque des revendications 5 à 7, dans lequel la quantité d'eau oxygénée est de 0,9 à 1,1 équivalent molaire par rapport à l'hespéridine engagée.

9. Procédé selon l'une quelconque des revendications 1 à 8, dans lequel le solvant aprotique polaire est le diméthylsulfoxyde.

10. Procédé selon l'une quelconque des revendications 1 à 9, dans lequel la diosmine obtenue est purifiée par un traitement base/acide.

11. Procédé selon l'une quelconque des revendications 1 à 9, dans lequel la diosmine obtenue est d'abord réempâtée dans un solvant organique tel que le diméthylsulfoxyde, la N-butylpyrrolidone, la pyridine, le sulfolane, le carbonate de diméthyle ou le carbonate de propylène, ou dans un mélange de solvant organique et d'eau tel qu'un mélange de pyridine et d'eau, puis filtrée et rincée par de l'eau, avant de subir un traitement base/acide.

12. Procédé selon l'une quelconque des revendications 1 à 11, dans lequel la diosmine obtenue est purifiée par un traitement base/acide en présence d'eau oxygénée.

## Patentansprüche

1. Verfahren zur Herstellung von Diosmin durch Oxidation von Hesperidin mit einem oxidierenden Ioddonorpaar bei einer Temperatur von 80 bis 120°C in einem Gemisch aus polarem aprotischem Lösungsmittel und Essigsäure, gefolgt von der Isolierung durch Zugabe von Wasser, Filtration, Spülen und Trocknen.

2. Verfahren nach Anspruch 1, wobei das erhaltene Diosmin in Form von Diosmin gemäß Pharmacopeia vorliegt, umfassend 90,0 bis 102,0% Diosmin, weniger als 4,0% Hesperidin, weniger als 2,0% Diosmetin, weniger als 3,0% Isorhoifolin, weniger als 3,0% Linarin und weniger als 0,6% 6-Ioddiosmin.

3. Verfahren nach Anspruch 1, wobei das erhaltene Diosmin in Form einer Flavonsäurefraktion vorliegt, umfassend 86,0 bis 95,0% Diosmin, 2,5 bis 6,0% Hesperidin, 0,2 bis 2% Diosmetin, 0,8 bis 3,5% Linarin und 0,8 bis 3,5% Isorhoifolin.

4. Verfahren nach Anspruch 1 oder 3, wobei das erhaltene Diosmin weniger als 0,6% 6-Ioddiosmin enthält.

5. Verfahren nach einem der Ansprüche 1 bis 4, wobei das oxidierende Ioddonorpaar aus NaI/H₂O₂, KI/H₂O₂, TBAI/H₂O₂ und NaI/I₂/H₂O₂ ausgewählt ist.

6. Verfahren nach Anspruch 5, wobei das oxidierende Ioddonorpaar NaI/H₂O₂ ist.

7. Verfahren nach Anspruch 6, wobei die Menge an Nal 0,4 bis 0,8 Moläquivalente bezogen auf das eingesetzte Hesperidin beträgt.

8. Verfahren nach einem der Ansprüche 5 bis 7, wobei die Menge an Wasserstoffperoxid 0,9 bis 1,1 Moläquivalente bezogen auf das eingesetzte Hesperidin beträgt.

9. Verfahren nach einem der Ansprüche 1 bis 8, wobei das polare aprotische Lösungsmittel Dimethylsulfoxid ist.

10. Verfahren nach einem der Ansprüche 1 bis 9, wobei das erhaltene Diosmin durch eine Base/Säure-Behandlung gereinigt wird.

11. Verfahren nach einem der Ansprüche 1 bis 9, wobei das erhaltene Diosmin zuerst in einem organischen Lösungsmittel wie Dimethylsulfoxid, N-Butylpyrrolidon, Pyridin, Sulfolan, Dimethylcarbonat oder Propylencarbonat oder in einem Gemisch aus organischem Lösungsmittel und Wasser wie einem Gemisch aus Pyridin und Wasser aufgeschlossen, dann filtriert und mit Wasser gespült wird, bevor es einer Base/Säure-Behandlung unterzogen wird.

12. Verfahren nach einem der Ansprüche 1 bis 11, wobei das erhaltene Diosmin durch eine Base/Säure-Behandlung in Gegenwart von Wasserstoffperoxid gereinigt wird.

## Claims

1. A method for the preparation of diosmin by oxidation of hesperidin by an iodine-donating oxidizing couple, at a temperature of 80 to 120°C, in a mixture of polar aprotic solvent and acetic acid, followed by isolation by addition of water, filtration, rinsing, and drying.

2. The method according to claim 1, wherein the diosmin obtained is in the form of Diosmin Pharmacopoeia, comprising from 90.0 to 102.0% of diosmin, less than 4.0% of hesperidin, less than 2.0% of diosmetin, less than 3.0% of isorhoifolin, less than 3.0% of linarin and less than 0.6% of 6-iododiosmin.

3. The method according to claim 1, wherein the diosmin obtained is in the form of a flavonoid fraction comprising from 86.0 to 95.0% of diosmin, from 2.5 to 6.0% of hesperidin, from 0.2 to 2% of diosmetin, from 0.8 to 3.5% of linarin and from 0.8 to 3.5% of isorhoifolin.

4. The method according to claim 1 or 3, wherein the diosmin obtained contains less than 0.6% of 6-iododiosmin.

5. The method according to any of claims 1 to 4, wherein the iodine-donating oxidizing couple is selected from NaI/H₂O₂, KI/H₂O₂, TBAI/H₂O₂, and NaI/I₂/H₂O₂.

6. The method according to claim 5, wherein the iodine-donating oxidizing couple is NaI/H₂O₂.

7. The method according to claim 6, wherein the amount of NaI is from 0.4 to 0.8 molar equivalents relative to the hesperidin used.

8. The method according to any of claims 5 to 7, wherein the quantity of hydrogen peroxide is from 0.9 to 1.1 molar equivalents relative to the hesperidin used.

9. The method according to any of claims 1 to 8, wherein the polar aprotic solvent is dimethyl sulfoxide.

10. The method according to any of claims 1 to 9, wherein the diosmin obtained is purified by an acid/base treatment.

11. The method according to any of claims 1 to 9, wherein the diosmin obtained is first re-pasted into an organic solvent such as dimethyl sulfoxide, N-butylpyrrolidone, pyridine, sulfolane, dimethyl carbonate, or propylene carbonate, or in a mixture of an organic solvent and water, such as a mixture of pyridine and water, then filtered and rinsed with water, before undergoing an acid/base treatment.

12. The method according to any of claims 1 to 11, wherein the diosmin obtained is purified by an acid/base treatment.
